# EUROPEAN PATENT SPECIFICATION

(11) **EP 4 081 190 B1**
(45) Date of publication and mention of the grant of the patent: **07.02.2024**
(21) Application number: 21719963.7
(22) Date of filing: 23.03.2021
(51) Int. Cl.: A61K 9/02, A61P 13/08, A61K 31/19, A61K 31/728, A61K 47/10, A61K 47/26, A61K 47/44

(54) **COMPOSITION FOR SUPPOSITORIES FOR THE TREATMENT OF THE PROSTATIC DISEASES**
ZUSAMMENSETZUNG FÜR ZÄPFCHEN ZUR BEHANDLUNG DER PROSTATERKRANKUNGEN
COMPOSITION POUR SUPPOSITOIRES POUR LE TRAITEMENT DES MALADIES PROSTATIQUES

(30) Priority: 07.04.2020 IT 202000007423
(43) Date of publication of application: 02.11.2022
(73) Proprietor: CAPRIKA SRL, 41012 Carpi (MO) (IT)
(72) Inventor: SALVATORE, Cristian, 80014 Giugliano in Campania (NA) (IT); DANZO, Nicola, 80014 Giugliano in Campania (NA) (IT)
(74) Representative: Studio Torta S.p.A.
(86) International application number: PCT/IB2021/052401
(87) International publication number: WO 2021/205268

(56) References cited:
- WO-A1-98/40064
- CN-B- 101 292 969
- R KUEFER ET AL: "Sodium butyrate and tributyrin induce in vivo growth inhibition and apoptosis in human prostate cancer", BRITISH JOURNAL OF CANCER, vol. 90, no. 2, 1 January 2004 (2004-01-01), pages 535-541, XP055757668, GB ISSN: 0007-0920, DOI: 10.1038/sj.bjc.6601510

## Description

### CROSS-REFERENCE TO RELATED APPLICATIONS

This patent application claims priority from Italian patent application no. 102020000007423 filed on 07/04/2020.

### TECHNICAL FIELD

The present invention relates to a composition for suppositories for use in the treatment of prostatitis and of benign prostatic hyperplasia.

### BACKGROUND ART

Inflammations of the lower urinary tract, in particular prostatitis, are very frequent in the male population and the symptoms that mostly characterize this disease are pain and erectile dysfunction. Subsequent to classification implemented in 1999 by the National Institute of Health, there are different categories of prostatitis, which mainly occur in chronic form with pain in the pelvic area due to the ongoing inflammatory process.

The treatment commonly used is based on the administration of antibiotics capable of eradicating the pathogen that causes the disease and on the use of drugs able to alleviate painful symptoms, such as NSAIDs, alpha blockers, 5-alpha-reductase inhibitors and phytotherapeutic preparations.

The use of oral anti-inflammatory drugs is not indicated as the first choice due to their toxicity, in particular with regard to cortisone anti-inflammatory drugs. Cortisones are anti-inflammatory and immunosuppressant drugs with a structure similar to endogenous corticosteroids. Although cortisone drugs have a high anti-inflammatory activity, their oral administration is not recommended due to their high systemic toxicity.

In this regard, second generation cortisone drugs, such as budesonide and the beclomethasone dipropionate which have reduced systemic toxicity, have been introduced for rectal use. In fact, the use of topical anti-inflammatory treatments is recommended both for the rapidity of administration and for the reduction in systemic toxicity.

However, it has been found experimentally that second generation cortisone drugs, even if administered rectally, still have some important side effects: nausea, headaches, local irritation and itching. The most important side effect is a reduction of cortisol levels followed by leucocytosis, morning facial swelling and visual problems. Patent document WO9840064 discloses rectal administration of butyric acid prodrugs for the treatment of prostate cancer.

Therefore, to treat prostatitis, the need is felt to provide a composition for rectal use that has effective anti-inflammatory activity and that, at the same time, does not cause the side effects of the above-mentioned prior art.

The inventors of the present invention have manufactured a composition for rectal use based on a salt of butyric acid capable of satisfying the aforesaid requirement.

The composition object of the present invention has proved to be effective both for the treatment of prostatitis and for the treatment of benign prostatic hyperplasia, which is also a disease associated with inflammation of the prostate.

### DISCLOSURE OF INVENTION

The object of the present invention is a suppository composition for use in the treatment of prostatitis and/or of benign prostatic hyperplasia; said composition being characterized in that it comprises 0.2% to 0.8% by weight of sodium hyaluronate butyrate, equal or more than 55.5% by weight of a waxy structural system and 11% to 33% by weight of an emulsifier system designed to incorporate said sodium hyaluronate butyrate.

Here and hereinafter, waxy structural system is meant as a complex of excipients with a waxy consistency capable of giving the suppository a solid structure at room temperature, so that it is sufficiently rigid to be administered, but at the same time allows the suppository to melt at body temperature (melting at rectal conditions: 2-3 ml of viscous aqueous secretion at 37°C) .

Here and hereinafter, emulsifier system is meant as a complex of excipients comprising at least one surfactant and designed to produce a "reservoir" system in which the salt of butyric acid is incorporated and to determine a controlled release of the salt of butyric acid.

Sodium hyaluronate butyrate is a derivative of the sodium salt of hyaluronic acid, in which some hydroxyl groups are bonded with ester linkages to the butyric acid. This compound has a high solubility in water and an increased lipophilia compared to sodium hyaluronate.

Preferably, said salt of butyric acid is sodium hyaluronate butyrate.

Preferably, said waxy structural system comprises a compound chosen between glycol distearate and polyethylene glycol. More preferably, said waxy structural system comprises a complex of two types of polyethylene glycol with a different molecular weight; even more preferably, it comprises a first polyethylene glycol with a mean molecular weight ranging from 1000 to 2000 and a second polyethylene glycol with a mean molecular weight ranging from 3000 to 6000.

Preferably, said waxy structural system further comprises a compound chosen between cetyl alcohol and Ceteareth-25.

Preferably, said waxy structural system comprises 20 to 35 % by weight of said first polyethylene glycol, 25 to 40 % by weight of said second polyethylene glycol and 15 to 25 % by weight of said cetyl alcohol.

Preferably, said emulsifier system comprises a combination of a first compound which is chosen from among Polysorbate 61 (TWEEN 61), Sorbitan Stearate (SPAN 60), PEG-100 Stearate, Glyceryl Stearate and Polysorbate 60 and of a second compound which is chosen from among Butylene glycol, Glycerin and Propylene glycol. In the emulsifier system the first and second compounds are in a weight ratio ranging from 0.5 to 1.5.

Preferably, said composition for suppositories comprises 28.0% by weight of PEG 4000, 25.5% by weight of PEG 1500, 19.1% by weight of cetyl alcohol, 12.0% by weight of Polysorbate 60, 10.0% by weight of propylene glycol, 5.0% by weight of purified water and 0.4% by weight of sodium hyaluronate butyrate.

Another object of the present invention is a suppository for the treatment of prostatitis and/or of benign prostatic hyperplasia characterized in that it is manufactured with the composition according to the present invention.

### BEST MODE FOR CARRYING OUT THE INVENTION

A description of an example is provided below for non-limiting illustrative purposes to offer a better understanding of the present invention.

A suppository was prepared with the formulation set down in Table I, in which the different ingredients and the relative amounts expressed as % by weight are indicated.

**Table I**

| Ingredient | % by weight |
|---|---|
| PEG 4000 | 28.0 |
| PEG 1500 | 25.5 |
| Cetyl alcohol | 19.1 |
| Polysorbate 60 | 12.0 |
| Propylene glycol | 10.0 |
| Purified water | 5.0 |
| Sodium hyaluronate butyrate | 0.4 |

The PEG 4000 is marketed by the company BRENNTAG.

The PEG 1500 is marketed by the company A.C.E.F..

The Sodium hyaluronate butyrate is marketed with the name ITHABUT^{®}.

The procedure used to manufacture the suppository is set down below.
- Preparation of the fatty mass: PEG 4000, PEG 1500 and cetyl alcohol were poured into a melting container where they were stirred at a temperature of around 80 °C, until obtaining a homogeneous molten mass; without interrupting stirring, the homogeneous molten mass produced as above was left to cool until reaching a temperature of around 50°C.
- Preparation of the gel: separately, and without using heat, the gel of sodium hyaluronate butyrate was prepared by adding the whole of the required amount of sodium hyaluronate butyrate to the water, under fast stirring.
- Introduction of the emulsifiers: propylene glycol and Polysorbate 60 were added to the fatty mass, which was previously melted at a temperature of around 40 °C.
- Combination of the phases: the sodium hyaluronate butyrate gel was added to the fatty mass with the emulsifier added and the system produced was stirred constantly and at a temperature of around 50°C.

The excipients used to produce the suppositories have a constitutive, productive, release and/or preservative role, and must ensure that the suppository remains solid at room temperature, so that it is sufficiently rigid to be administered and that, at the same time, it is capable of melting at body temperature (melting at rectal conditions: 2-3 ml of viscous aqueous secretion at 37°C).

In this regard, a water-soluble base excipient like polyethylene glycol was chosen.

A fundamental feature of water-soluble bases is that they can melt at a temperature above rectal temperature but require first to be dissolved or dispersed in biological fluids (rectal mucus) .

In water-soluble masses the active ingredient is released through dissolution of the excipient in the rectum; the initial concentrated solution attracts water through the rectal wall helping to maintain the dispersion of the active ingredient inside the suppository homogeneous.

The use of blends of polyethylene glycols with different MW (from 1000 to 6000) allows the composition to be modulated for it to be given the desired properties: PEGs with high molecular weight slow down dissolution.

The rate-determining step is represented by distribution of the drug between excipient and rectal mucus: hydrophilic drugs will be released more rapidly by lipophilic excipients; on the contrary, lipophilic drugs will be spread more rapidly by hydrophilic excipients. Therefore, given the hydrophilic nature of the active ingredient, in this case slow release is obtained.

Cetyl alcohol is an aliphatic long chain saturated fatty alcohol (16 carbon atoms) and represents a waxy consistency factor mainly used to increase the melting temperature of the suppository; it also contributes to the stability of the emulsion by mixing with the hydrophilic emulsifying agent.

In this regard, other excipients that can be added to the formulation of suppositories comprise adsorbents, surfactants, lubricants, emulsifiers.

From a chemical-technological viewpoint, the emulsions fall within the category of disperse systems. The term "dispersion" indicates all those systems formed of a substance (liquid, solid or gaseous) dispersed in a second substance (generally liquid or gaseous) which, combined in variable proportions, generally appear turbid and more or less fluid.

The surfactant (Polysorbate 60) is capable of self-emulsifying forming O/W emulsions. This property accelerates breakdown of the suppository in the rectum and hence absorption of the active ingredient.

The emulsifier system formed of Polysorbate 60 and Propylene glycol is extremely important not only for its role of controlled release factor of the active ingredient of the emulsion, but also for the function of "reservoir" system in which Sodium hyaluronate butyrate is incorporated.

As will be illustrated below, an experimental study has confirmed that the suppositories as manufactured above based on sodium hyaluronate butyrate guarantee the effectiveness of a rapid topical and low toxicity treatment. In this regard, the inventors of the present invention have surprisingly found that sodium hyaluronate butyrate has an important anti-inflammatory activity when administered topically and has no known side-effects. Prior to the present invention, the sodium hyaluronate butyrate was used for completely different purposes, such as in the cosmetics sector with the advantage of promoting an increase in the elasticity of the skin.

It is also important to note that, when sodium hyaluronate butyrate is used in combination with propylene glycol, its filmogenic activity is exploited to increase the contact of said sodium hyaluronate butyrate with the prostate.

### EXPERIMENTAL STEP

A treatment was tested on a group of twenty patients ranging from 18 to 70 years in age and suffering from prior and full-blown acute prostatitis. Patients with chronic liver and kidney diseases, metabolic, neurological or gastrointestinal (constipation or chronic diarrhoea) disorders, prior prostate surgery, urethral stricture or bladder neck sclerosis were excluded.

The patients were treated with suppositories as described above, with one daily application for twenty days. Observation was carried out on all the patients ten days after the end of administration.

The observation included IPSS score (scale from 1 to 35) and VAS scores (scale from 1 to 8). Moreover, PSA was tested, also ten days after the end of administration.

In particular, in the VAS score the following symptoms were measured: irritation and suprapubic pain; perineal pain; burning/pain during urination; urgent or frequent need to pass urine; feeling of incomplete emptying.

Table I indicates, for each analysis parameter, the mean values obtained for the twenty patients at the start of and after treatment.

**Table I -**

| Before treatment | After treatment |
|---|---|
| IPSS score (scale 1 - 35) | |
| 24 | 11 |
| Irritation and suprapubic pain (scale 1 - 8) | |
| 5 | 3.5 |
| Perineal pain (scale 1 - 8) | |
| 4 | 3 |
| Burning/pain during urination (scale 1 - 8) | |
| 5 | 3 |
| Urgent or frequent need to pass urine (scale 1 - 8) | |
| 7 | 5 |
| Feeling of incomplete emptying (scale 1 - 8) | |
| 6 | 4 |
| PSA (ng/ml) | |
| 3.5 | 2.8 |

The considerable improvements in terms of symptomatic parameters relating to prostatitis following treatment with the suppositories of the present invention are evident in Table I.

Moreover, it is extremely relevant to point out that, in terms of side effects, no clinically significant phenomena related to the treatment were found and that, consequently, the very high tolerability of the components of the formulation of the suppositories of the invention can be confirmed. In conclusion, the inventors of the present invention, having verified the unexpected effects of sodium hyaluronate butyrate in the treatment of prostatitis and/or of prostatic hyperplasia, have produced an extremely effective topical treatment (suppository), without causing the normal side effects of treatments.

## Claims

1. A suppository composition for use in the treatment of prostatitis and/or benign prostatic hyperplasia; said composition being **characterized in that** it comprises 0.2% to 0.8% by weight of sodium hyaluronate butyrate, equal or more than 55.5% by weight of a waxy structural system and 11% to 33% by weight of an emulsifier system designed to incorporate said sodium hyaluronate butyrate.

2. A suppository composition for use according to claim 1, **characterized in that** said waxy structural system comprises a compound chosen between glycol distearate and polyethylene glycol.

3. A suppository composition for use according to claim 2, **characterized in that** said waxy structural system comprises a complex of two types of polyethylene glycol with a different molecular weight.

4. A suppository composition for use according to claim 3, **characterized in that** said waxy structural system comprises a first polyethylene glycol with a mean molecular weight ranging from 1000 to 2000 and a second polyethylene glycol with a mean molecular weight ranging from 3000 to 6000.

5. A suppository composition for use according to claim 3 or 4, **characterized in that** said waxy structural system comprises a compound chosen between cetyl alcohol and Ceteareth-25.

6. A suppository composition for use according to claims 4 or 5, **characterized in that** said waxy structural system comprises 20 to 35 % by weight of said first polyethylene glycol, 25 to 40 % by weight of said second polyethylene glycol and 15 to 25 % by weight of said cetyl alcohol.

7. A suppository composition for use according to one of the preceding claims, **characterized in that** said emulsifier system comprises a combination of a first compound, which is chosen among Polysorbate 61 (TWEEN 61), Sorbitan Stearate (SPAN 60), PEG-100 Stearate, Glyceryl Stearate and polysorbate 60, and of a second compound, which is chosen among butylene glycol, glycerin and propylene glycol; said first and said second compounds being available in a weight ratio ranging from 0.5 to 1.5.

8. A suppository composition for use according to one of the preceding claims, **characterized in that** said composition for suppositories comprises 28.0% by weight of PEG 4000, 25.5% by weight of PEG 1500, 19.1% by weight of cetyl alcohol, 12.0% by weight of polysorbate 60, 10.0% by weight of propylene glycol, 5.0% by weight of purified water and 0.4% by weight of sodium hyaluronate butyrate.

9. A suppository for use in the treatment of prostatitis and/or benign prostatic hyperplasia, **characterized in that** it is manufactured with the composition according to one of the preceding claims.

## Patentansprüche

1. Suppositoriumszusammensetzung zur Verwendung in der Behandlung von Prostatitis und/oder benigner Prostatahyperplasie; wobei die Zusammensetzung **dadurch gekennzeichnet ist, dass** sie 0,2 bis 0,8 Gew.-% Natriumhyaluronat-Butyrat, gleich oder mehr als 55,5 Gew.-% eines wachsartigen Struktursystems und 11 bis 33 Gew.-% eines Emulgatorsystems umfasst, das dazu bestimmt ist, das Natriumhyaluronat-Butyrat einzubinden.

2. Suppositoriumszusammensetzung zur Verwendung nach Anspruch 1, **dadurch gekennzeichnet, dass** das wachsartige Struktursystem eine Verbindung umfasst, die aus Glykoldistearat und Polyethylenglykol ausgewählt ist.

3. Suppositoriumszusammensetzung zur Verwendung nach Anspruch 2, **dadurch gekennzeichnet, dass** das wachsartige Struktursystem einen Komplex aus zwei Arten von Polyethylenglykol mit einem unterschiedlichen Molekulargewicht umfasst.

4. Suppositoriumszusammensetzung zur Verwendung nach Anspruch 3, **dadurch gekennzeichnet, dass** das wachsartige Struktursystem ein erstes Polyethylenglykol mit einem mittleren Molekulargewicht im Bereich von 1000 bis 2000 und ein zweites Polyethylenglykol mit einem mittleren Molekulargewicht im Bereich von 3000 bis 6000 umfasst.

5. Suppositoriumszusammensetzung zur Verwendung nach Anspruch 3 oder 4, **dadurch gekennzeichnet, dass** das wachsartige Struktursystem eine Verbindung umfasst, die aus Cetylalkohol und Ceteareth-25 ausgewählt ist.

6. Suppositoriumszusammensetzung zur Verwendung nach Anspruch 4 oder 5, **dadurch gekennzeichnet, dass** das wachsartige Struktursystem 20 bis 35 Gew.-% des erstes Polyethylenglykols, 25 bis 40 Gew.-% des zweiten Polyethylenglykols und 15 bis 25 Gew.-% des Cetylalkohols umfasst.

7. Suppositoriumszusammensetzung zur Verwendung nach einem der vorhergehenden Ansprüche, **dadurch gekennzeichnet, dass** das Emulgatorsystem eine Kombination einer ersten Verbindung, die aus Polysorbat 61 (TWEEN 61), Sorbitanstearat (SPAN 60), PEG-100 Stearat, Glycerolstearat und Polysorbat 60 ausgewählt ist und einer zweiten Verbindung, die zwischen Butylenglykol, Glycerin und Propylenglykol ausgewählt ist, umfasst; wobei die erste und die zweite Verbindung in einem Gewichtsverhältnis im Bereich von 0,5 bis 1,5 vorliegen.

8. Suppositoriumszusammensetzung zur Verwendung nach einem der vorhergehenden Ansprüche, **dadurch gekennzeichnet** das die Zusammensetzung für Suppositorien 28,0 Gew.-% PEG 4000, 25,5 Gew.-% PEG 1500, 19,1 Gew.-% Cetlyalkohol, 12,0 Gew.-% Polysorbat 60, 10,0 Gew.-% Propylenglykol, 5,0 Gew.-% gereinigtes Wasser und 0,4 Gew.-% Natriumhyaluronat-Butyrat umfasst.

9. Suppositorium zur Verwendung in der Behandlung von Prostatitis und/oder benigner Prostatahyperplasie, **dadurch gekennzeichnet, dass** dieses mit der Zusammensetzung nach einem der vorhergehenden Ansprüche hergestellt ist.

## Revendications

1. Composition suppositoire pour le traitement de la prostatite et/ou de l'hyperplasie bénigne de la prostate ; cette composition est **caractérisée par le fait qu'**elle comprend de 0,2 % à 0,8 % en poids de butyrate de hyaluronate de sodium, au moins 55,5 % en poids d'un système structurel cireux et de 11 % à 33 % en poids d'un système émulsifiant conçu pour incorporer le butyrate de hyaluronate de sodium.

2. Composition suppositoire selon la revendication 1, **caractérisée par le fait que** le système structurel cireux comprend un composé choisi entre le distéarate de glycol et le polyéthylène glycol.

3. Composition suppositoire selon la revendication 2, **caractérisée par le fait que** le système structurel cireux comprend un complexe de deux types de polyéthylène glycol de poids moléculaire différent.

4. Composition suppositoire selon la revendication 3, **caractérisée par le fait que** le système structurel cireux comprend un premier polyéthylène glycol dont le poids moléculaire moyen est compris entre 1 000 et 2 000 et un second polyéthylène glycol dont le poids moléculaire moyen est compris entre 3 000 et 6 000.

5. Composition suppositoire à utiliser selon la revendication 3 ou la revendication 4, **caractérisée par le fait que** le système structurel cireux comprend un composé choisi entre l'alcool cétylique et le Ceteareth-25.

6. Composition suppositoire à utiliser selon les revendications 4 ou 5, **caractérisée en ce que** ledit système structurel cireux comprend de 20 à 35 % en poids dudit premier polyéthylène glycol, de 25 à 40 % en poids dudit second polyéthylène glycol et de 15 à 25 % en poids dudit alcool cétylique.

7. Composition pour suppositoire à utiliser selon l'une des revendications précédentes, **caractérisée en ce que** ledit système émulsifiant comprend une combinaison d'un premier composé, qui est choisi parmi le polysorbate 61 (TWEEN 61), le stéarate de sorbitan (SPAN 60), le stéarate de PEG-100, le stéarate de glycéryle et le polysorbate 60, et d'un second composé, qui est choisi parmi le butylène glycol, la glycérine et le propylène glycol ; lesdits premier et second composés étant disponibles dans un rapport de poids allant de 0,5 à 1,5.

8. Composition pour suppositoires à utiliser selon l'une des revendications précédentes, **caractérisée en ce que** ladite composition pour suppositoires comprend 28,0 % en poids de PEG 4000, 25,5 % en poids de PEG 1500, 19,1 % en poids d'alcool cétylique, 12,0 % en poids de polysorbate 60, 10,0 % en poids de propylène glycol, 5,0 % en poids d'eau purifiée et 0,4 % en poids de butyrate d'hyaluronate de sodium.

9. Suppositoire pour le traitement de la prostatite et/ou de l'hyperplasie bénigne de la prostate, **caractérisé par le fait qu'**il est fabriqué avec la composition selon l'une des revendications précédentes.
